# EUROPEAN PATENT APPLICATION

(11) **EP 1 782 838 A1**
(43) Date of publication of application: **09.05.2007**
(21) Application number: 05753336.6
(22) Date of filing: 23.06.2005
(51) Int. Cl.: A61K 48/00, A61K 31/702, A61K 38/46, A61K 39/395, A61P 35/00, A61P 43/00

(54) **INHIBITION OF INFILTRATION, AND CELL KILLING AGENT**

(30) Priority: 23.06.2004 JP 2004184583
(71) Applicant: Japan Science and Technology Agency, Kawaguchi-shi, Saitama 332-0012 (JP)
(72) Inventor: NAKAMURA, Mitsuru; c/o National Institute, 1-chome Ibaraki 305-0045 (JP); KIKUCHI, Jiro; National Institute, Tsukuba-shi ibaraki 305-0045 (JP); NONOMURA, Chizu; c/o Japan Science and, Kawaguchi-shi Saitam a; 3320012 (JP); ANDO, Hidenobu; c/o National Institute, Tsukuba-shiIbaraki, .05-0045 (JP)
(74) Representative: Leidescher, Thomas
(86) International application number: PCT/JP2005/011560
(87) International publication number: WO 2006/001348

(57) **Abstract**

As suppressor of binding between selectin binding sugar chain and selectin or cell killing agent, (1) siRNA suppressing expression of genes which relates to cell surface antigen CD43 and selectin ligand sugar chain by RNA interference; (2) an inhibitor of sugar chain synthesis such as analogue of substrates in sugar chain synthesis; (3) an enzyme severing cell surface antigen CD43 and selectin ligand sugar chain; and (4) antibodies of cell surface antigen CD43 and selectin ligand sugar chain are used to selectin ligand sugar chain expressed in cell surface antigen CD43 to provide a suppressor of infiltration which is able to suppression of infiltration to tissues by leukocytes and a method of suppression thereof, and a cell killing agent of CD43 expressing cell and a cell killing method thereof.

## Description

### Technical Field

The present invention relates to a suppressor of infiltration of malignant tumor cells to tissue and a cell killing agent, particularly, to an infiltration suppressor of binding of a selectin binding sugar chain characteristically expressed on a cell surface protein to selectin and a cell killing agent targeting the characteristic selectin binding sugar chain and a glycoprotein.

### Related Art

One of the important issues of malignant tumors is metastasis and infiltration of tumor cells to tissue and organs. More specifically, in the case where malignant tumor cells infiltrate and metastasize to an important organ, dysfunction occurs in such organ to result in disturbance in the whole living body. Also, intercell signaling due to adhesion of tumor cells to vascular endothelial cells induce resistance to an anticancer drug to result in deterioration of therapeutic effect (Non-Patent Publication 1).

Therefore, the suppression of the malignant tumor cell adhesion and the suppression of the tissue/organ infiltration/metastasis due to the adhesion have important significance in therapy. However, a current therapeutic method for the organ infiltration and metastasis is at a stage of clinical tests of an angiogenesis suppressor or an antibody for a growth factor as a therapeutic drug and a therapeutic drug or method for directly suppressing the intercellular adhesion that is the cause of the infiltration and induces tolerance to anticancer drugs have not come into practical use.

On the other hand, it has been reported that an antigen that is not observed in normal cells appears on surfaces of cancer cells when cells become cancerous (Non-Patent Publication 2). It has been proved that many of the cancer-associated antigens are carbohydrate antigens. The carbohydrate antigens actively change along with differentiation, canceration, and leukosis of epidermal cells or white blood cells (Non-Patent Publications 3 and 4), and a part thereof is used for a cancer test as a tumor marker (Non-Patent Publication 5). Further, these carbohydrate antigens appearing in cancer have a significant function, and it has been reported that a part of them has a strong cell adhesion activity. Among the carbohydrate antigens, it has been reported that the tumor marker CA19-9 is a molecule having a function of causing cancer cells in digestive system to adhere blood vessels (Non-Patent Publication 6). A sugar chain to be detected by the tumor marker CA19-9 is sialyl Lewis a, and it has been reported that sialyl Lewis a binds to E-selectin which is a cell adhesion molecule of the vascular endothelial cells with sialyl Lewis x. From the above findings, it is suggested that the sugar chain (selectin ligand sugar chain) molecule has the function of promoting acquisition of anticancer agent tolerance and vascular infiltration and hematogenous metastasis of cancer via the adhesion of cancer cells to capillary endothecium. Further, it has been reported that, in patients who have cancer cells that strongly expresses the sugar chains such as sialyl Lewis a and sialyl Lewis x, the hematogenous metastasis is caused quickly, and prognosis is worse when compared with patients without such cancer cells.

The selectin ligand sugar chain is expressed also in acute T lymphocytic leukemic cells, and correlativity between the selectin ligand sugar chain and skin infiltration at the expression level has been found (Non-Patent Publication 7). From the above findings, involvement of action mediated by the binding of the selectin ligand sugar chain to selectin in the course of cell adhesion and tissue infiltration of leukemic cells is strongly suggested. On the other hand, organ infiltration of the leukemic cells is also observed in acute pre-B lymphocytic leukemia (Non-Patent Publication 8), and it has been reported that transplantation of pre-B lymphocytic leukemia cells into an immunodeficient mouse induces infiltration to various organs including bone marrow and abnormal growth of leukemic cells to result in death of the mouse (Non-Patent Publication 9). However, it has been unknown that the selectin ligand sugar chains are expressed in the pre-B lymphocytic leukemia cells; most of major selectin ligand sugar chains are expressed on cell surface antigen CD43 glycoproteins; and the selectin ligand sugar chains on the CD43 antigen have a predominant role in the tissue infiltration.
Non-Patent Publication 1: Nat. Med., 9, 1158-1165 (2003)
Non-Patent Publication 2: Natl. Cancer Inst., 71, 231-251 (1983)
Non-Patent Publication 3: Proc. Natl. Acad. Sci. USA, 80, 2833-2848 (1983)
Non-Patent Publication 4: Science, 220, 509-511 (1983)
Non-Patent Publication 5: Cancer Res., 48, 3856-3863 (1988)
Non-Patent Publication 6: Cancer Res., 53, 354-361 (1993)
Non-Patent Publication 7: Cancer Res., 55, 6533-6538 (1994)
Non-Patent Publication 8: Blood, 88, 1135-1140 (1996)
Non-Patent Publication 9: Blood, 78, 2973-2981 (1991)

### Disclosure of the Invention

### Problems to be Solved by the Invention

In a malignant tumor, particularly in a hematopoietic malignant tumor such as leukemia and malignant lymphoma, adhesion of tumor cells to vascular endothelial cells and tissue infiltration via the adhesion are the causes of suppression of cell killing effect by anticancer agents and cancer recurrence which are problems in therapy. Particularly, infiltration to central nervous tissue to which it is difficult to exhibit effects of the anticancer agents and radiation therapy is a serious problem in definitive therapy of leukemia. Therefore, suppression of the cell adhesion and the organ infiltration via the adhesion is an important issue in therapy of the hematopoietic malignant tumor. However, the conventional therapeutic agents for suppressing the cell adhesion and the tissue infiltration/metastasis have not been satisfactory, and there has been a demand for development of an agent capable of more effectively suppressing the cell adhesion and the tissue infiltration/metastasis.

The inventors of the present invention have conducted extensive researches in order to realize the suppression of cell adhesion and tissue infiltration of tumor cells in malignant tumor, particularly in hematopoietic malignant tumor, to find that a selectin binding sugar chain is locally present in a cell surface antigen CD43 and that intercell adhesion occurs due to binding of tumor cells to selectin in infiltrated tissues mediated by the selectin binding sugar chain to develop infiltration/metastasis through the adhesion process. Further, the inventors have found that it is possible to suppress the cell adhesion and the tissue infiltration/metastasis of tumor cell by suppressing expression of the CD43 antigen and the selectin ligand to produce a suppressor of the expressions of CD43 antigen and selectin ligand sugar chain and a selectin binding inhibitor as tissue infiltration suppressor as well as a substance exhibiting a binding activity on the selectin ligand sugar chain and its carrier protein CD43 as a cell killing agent, thereby accomplishing the present invention.

More specifically, the present invention relates to the following (1) to (27).
(1) An infiltration suppressor which suppresses binding between a selectin ligand sugar chain on a cell surface and selectin.
(2) The infiltration suppressor according to (1), which comprises a nucleic acid comprising a small-interfering RNA (siRNA) sequence as an active ingredient.
(3) The infiltration suppressor according to (2), wherein the small-interfering RNA (siRNA) sequence comprises any one of the nucleotide sequences of SEQ ID NOs:1 to 20.
(4) The infiltration suppressor according to (1), which comprises endo-O-glycosidase as an active ingredient.
(5) The infiltration suppressor according to (1), which comprises an anti-CD43 antibody as an active ingredient.
(6) The infiltration suppressor according to (1), which comprises at least one sugar chain selected from the group consisting of sugar chains having human-Fc-Ig-chimera E-selectin, human-Fc-Ig-chimera P-selectin, and a sialyl Lewis x structure as an active ingredient.
(7) Use of the infiltration suppressor according to any one of (1) to (6) for preventing tissue infiltration of a cell.
(8) An infiltration suppressing method, which comprises suppressing binding between a selectin ligand sugar chain on cell surface and selectin.
(9) The method according to (8), which comprises suppressing addition of the selectin ligand sugar chain to a cell surface antigen CD43.
(10) The method according to (8), which comprises suppressing expression of the cell surface antigen CD43.
(11) The method according to (8), which comprises suppressing expression of a glycosyltransferase gene adding the selectin ligand sugar chain to the cell surface antigen CD43.
(12) The method according to item (11), wherein the glycosyltransferase gene is a gene encoding an N-acetylgalactosaminyltransferase, a gene encoding a galactosyltransferase, a gene encoding an N-acetylglucosaminyltransferase, a gene encoding a fucosyltransferase, a gene encoding a sialyltransferase, or a gene encoding a sulfotransferase.
(13) The method according to any one of (8) to (12), which comprises using a nucleic acid comprising a small-interfering RNA (siRNA) sequence.
(14) The method according to (13), wherein the small-interfering RNA (siRNA) sequence comprises any one of the nucleotide sequences of SEQ ID NOs:1 to 20.
(15) The method according to (8), which comprises severing the binding between the cell surface antigen CD43 and the selectin ligand sugar chain.
(16) The method according to (8), which comprises decomposing the cell surface antigen CD43.
(17) The method according to (8), which comprises decomposing the selectin ligand sugar chain.
(18) Use of a nucleic acid comprising a small-interfering RNA (siRNA) sequence for suppressing of binding between a selectin ligand sugar chain and selectin.
(19) A cell killing agent which binds to a selectin ligand sugar chain on cell surface and a carrier protein retaining the sugar chain to induce cell death specifically on cells expressing a glycoprotein.
(20) The cell killing agent according to (19), wherein the carrier protein retaining the selectin ligand sugar chain is a CD43 protein.
(21) The cell killing agent according to (19), wherein a substance binding to the carrier protein is an antibody or a peptide specifically binding to an epitope in an extracellular region of the CD43 protein.
(22) The cell killing agent according to (21), to which a cytotoxic agent is bound.
(23) The cell killing agent according to (22), wherein the cytotoxic agent is selected from the group consisting of antimetabolites, alkylation agents, anthracyclines, antibiotics, antimitotic agents, chemotherapeutic agents, and radioactive substances.
(24) The cell killing agent according to (22), wherein the cytotoxic agent is selected from the group consisting of ricin, doxorubicin, daunorubicin, taxol, ethidium bromide, mitomycin, etoposide, tenoposide, vincristine, vinblastine, corticin, dihydroxyanthracindione, actinomycin D, 1-dehydrotestosterone, and glucocorticoid.
(25) The cell killing agent according to (21), wherein the antibody specifically binding to the epitope in the extracellular region of the CD43 protein is a human monoclonal antibody, a synthetic antibody, a polyclonal antibody, or a chimeric antibody.
(26) A cell killing method, which comprises binding the cell killing agent according to (19) to (25) to a selectin ligand sugar chain on a cell surface and a carrier protein retaining the sugar chain.
(27) The method according to (26), wherein the cell is hematopoietic lineage cell including a hematopoietic malignant tumor cell and a hematopoietic stem cell expressing CD43.

### Effect of the Invention

According to the present invention, agents for effectively suppressing intercell adhesion and tissue infiltration/metastasis, a method for suppressing the tissue infiltration/metastasis, agents for killing hematopoietic lineage cells including hematopoietic malignant tumor cells, and a method for the cell killing are provided.

### Best Mode for Carrying out the Invention

### 1. Agents of the present invention

The present invention relates to an infiltration suppressor which suppresses binding of a selectin ligand sugar chain on cell surface to selectin and to a cell killing agent which binds to the selectin ligand sugar chain on cell surface and a carrier protein retaining the sugar chain to induce cellular death specifically of cells expressing the glycoprotein. These agents are collectively referred to as "agents of the present invention".

The agents of the present invention is characterized by suppressing the binding of the selectin ligand on cell surface to selectin and by killing cells with targeting the carrier protein retaining the selectin ligand sugar chain.

The selectin ligand sugar chain on cell surface in the agents of the present invention means sialyl Lewis x, sulfosialyl Lewis x, and sialyl Lewis a and may most preferably be the sulfosialyl Lewis x or sialyl Lewis x.

Examples of the carrier protein retaining the selectin ligand sugar chain include a CD43 protein and the like, and a substance to bind to the carrier protein may preferably be an antibody or a peptide that specifically binds to an epitope in an extracellular region of the CD43 protein.

The sialyl Lewis x binds to the cell surface via the CD43. Therefore, in order to suppress the binding of sialyl Lewis x to selectin is achieved by (a) methods of suppressing the expression of CD43, (b) methods of severing or inhibiting the binding between CD43 and sialyl Lewis x, (c) methods of binding to a complex of CD43 and sialyl Lewis x to cause a displacement of configuration, (d) methods of specifically decomposing CD43 or sialyl Lewis x, (e) methods of competitive inhibition or rivalry inhibition of the binding between sialyl Lewis x and selectin, (f) methods of inhibiting synthesis of sialyl Lewis x, and the like. Also, cell killing which targets the selectin ligand sugar chain and its carrier protein CD43 is achieved by (g) induction of antibody dependent cell damaging activity by an anti-CD43 antibody, (h) an anti-CD43 antibody to which a cell killing agent such as an anticancer agent is bound, and the like.

Although cells to be the subject of the agents of the present invention are not particularly limited, insofar as the cells are tumor cells having property of infiltrating tissue, and the cells may preferably be hematopoietic malignant tumor cells, more preferably leukemic cells. Among the leukemic cells, tumor cells derived from B cell-derived cells may be preferred, and pre-B lymphocytic leukemic cells may particularly be preferred.

The infiltration suppression in the present invention means a prevention of a progress of infiltration of cells into tissue and prophylaxis of the infiltration of cells into tissue.
(a) As the methods for suppressing the expression of CD43, for example, a method of using a small-interfering RNA (hereinafter referred to as "siRNA") having a function of preventing normal expression of CD43 by causing RNA interference with a transcription product of a CD43 gene is preferred. Nucleotide sequences which have a function of expressing such siRNA in cells include nucleotide sequences comprising any of the nucleotide sequences of SEQ ID NOs:1 to 20, and a nucleotide sequence consisting of any of the nucleotide sequences of SEQ ID NOs:1 to 20 may particularly be preferred. However, the nucleotide sequence is not limited, insofar as it is capable of preventing the expression of CD43 based on RNA interference mechanism and may include other arbitrary nucleotide sequence. Such nucleotide sequence may be used as an active ingredient of the agents of the present invention.
   The agents of the present invention using the siRNA sequence as the active ingredient achieves the infiltration suppressing effect by introducing a nucleic acid prepared by an ordinary method into a virus vector by using a known methods such as a lentivirus system (product of Invitrogen Corporation) and then administering the virus vector to a living body to suppress the expression of CD43 antigen in subject cells.
(b) As the methods of severing or inhibiting the binding between CD43 and sialyl Lewis x, it is possible to sever a sugar chain including sialyl Lewis x from CD43 by using endo-O-glycosidase (product of ProZyme, Inc.) capable of dissociating an O-bound sugar chain from a core protein.
   The agents of the present invention using such enzyme as an active ingredient are capable of achieving a desired effect by an administration of the agents of the present invention to a living body in the form of an injectable solution.
(c) Examples of methods of binding to a complex of CD43 and sialyl Lewis x to cause a displacement of configuration include a method of using an antibody for sialyl Lewis x and CD43 and the like, for example. Examples of the antibody include a human monoclonal antibody, a synthetic antibody, a polyclonal antibody, a chimera antibody, and the like, and specific examples of the antibody include CSLEX (product of Becton, Dickinson and Company), KM93 antibody (product of SEIKAGAKU CORPORATION), DF-T1 antibody (product of Sigma-Aldrich Japan, K.K.), 1G10 antibody (product of Becton, Dickinson and Company), 1D4 antibody (product of MBL CO., LTD.), 84-3C1 antibody (product of NeoMarker), MEM59 antibody (product of Monosan), Bra7G antibody (product of NeoMarker), BL-E/G3 antibody (product of Monosan), MT-1 antibody (product of Progen GmbH), and the like, each of which is usable as an active ingredient of the agent of the present invention.
   It is possible to use a hybridoma for preparing the antibody for sialyl Lewis x and CD43 which is usable for the agent of the present invention. It is possible to produce such a hybridoma by a known cell fusion method. Namely, it is possible to obtain a desired hybridoma by immunizing an animal other than human by using a human CD43 antigen as an immunogen; producing hybridomas by fusing its spleen cells or lymph node cells with myeloma cells; and selecting one which produces a monoclonal antibody recognizing the human CD43 antigen among the hybridomas.
   Although the immunogen is not particularly limited, insofar as it contains CD43, and examples thereof include a grown matter (recombinant fragments, culture medium, excretory substances, secretions, and the like) obtainable by growing a recombinant produced by integrating a nucleic acid comprising a nucleotide sequence of a nucleic acid (J. Biol. Chem., 266, 13, 8483-8489 (1991)) encoding CD43 by an ordinary method or a CD43 antigen obtained by partially purifying or purifying the grown matter by affinity chromatography using an existing anti-CD43 antibody or the like and the CD43 antigen obtained by purification is particularly preferred.
   The animals to be immunized for preparing the hybridoma are not particularly limited, and examples thereof include a goat, a sheep, a guinea pig, a mouse, a rat, a rabbit, and the like. Among the above, the mouse is preferred.
   As the method for immunizing the animal, it is possible to employ a known method. In the case of immunizing a mouse, examples of the method include a method of emulsifying 1 to 100 µg, preferably 50 to 100 µg, of the immunizing antigen with a normal saline equal in quantity (0.1 mL) and a complete Freund's adjuvant system, an incomplete Freund's adjuvant system, or an RIBI adjuvant system and then inoculating the emulsion into the back, subdermal of the abdomen, or the abdominal cavity of the animal for 3 to 6 times per 2 to 3 weeks.
   After immunizing the animal, an individual having a high antibody titer is selected, and then the spleen or the lymph node is isolated after 3 to 5 days from the final immunization. It is possible to fuse antibody producing cells contained in tissue of the isolated spleen or lymph node with the myeloma cells under the presence of a fusion promoting agent by a known cell fusion method.
   The fusion promoting agent is not particularly limited. Examples of the fusion promoting agent include polyethylene glycol (hereinafter also referred to as "PEG"), Sendai virus, and the like, and it is preferable to use PEG.
   The myeloma cells are not particularly limited, and examples thereof include mouse-derived cells such as P3U1, NS-1, and P3x63.Ag8.653; rat-derived cells such as AG1 and AG2; and the like.
   The cell fusion method is not particularly limited, and examples thereof include a method of mixing the spleen cells with the myeloma cells at a ratio of 1:1 to 10:1, followed by addition of PEG having a molecular weight of from 1,000 to 6,000 at a concentration of 10% to 80%, and then incubating at 20°C to 37°C, preferably at 30°C to 37°C, for 3 to 10 minutes, and the like.
   In the agents of the present invention containing the anti-CD43 antibody as an active ingredient, the selection of hybridoma for producing the monoclonal antibody recognizing the CD43 antigen is performed through a culture in a selected culture medium such as a HAT culture medium in which only the hybridoma is able to grow and then measuring an antibody activity in a supernatant of the hybridoma culture by an ordinary method. Further, it is possible to establish the hybridoma for producing the monoclonal antibody specifically recognizing the CD43 antigen by repeating cloning by a method of limiting dilution of the hybridoma for producing the monoclonal antibody which specifically recognizes the CD43 antigen.
   The method of preparing the anti-CD43 antibody by a large scale is not particularly limited, and examples thereof include a method of implanting the hybridoma of the present invention in abdominal cavity of a mouse to which pristane has been administered and then obtaining the hybridoma from collected ascites. The antibody of the present invention in the ascites is purified easily by a known method using a protein A column, a protein G column, and the like.
   The agent of the present invention using the antibody as the active ingredient is able to achieve a desired effect when administered in the form of an injectable solution to a living body.
(d) Examples of the methods for specifically decomposing CD43 or sialyl Lewis x include an enzyme selectively decomposing a mucin type glycoprotein rich in sialic acid, an enzyme recognizing and severing a binding between specific sugar chains contained in the sialyl Lewis x structure, and the like. Examples of the enzyme include O-sialoglycoprotein endopeptidase (OSGPEP, product of Cedarlane Laboratories Ltd.), sialidase (product of ProZyme, Inc.) specifically severing α2,3-glycoside binding between sialic acid and galactose, β-galactosidase (product of ProZyme, Inc.) specifically severing β1,4-glycoside binding between galactose and N-acetylglucosamine, and the like, and it is possible to use any of the above means as an active ingredient of the agent of the present invention.
   The agents of the present invention using the enzyme as an active ingredient achieve a desired effect when administered to a living body in the form of an injectable solution or the like.
(e) Examples of the methods for competitively inhibiting or rivalry inhibiting the binding between sialyl Lewis x and selectin include a method of using a soluble recombinant selectin or a soluble sugar chain and the like. Examples of the soluble recombinant selectin include human Fc-Ig-chimera E-selectin and human Fc-Ig-chimera P-selectin (product of R & D systems), a sugar chain (product of Takara Bio Inc. and Seikagaku Corporation) having the sialyl Lewis x structure, and the like. It is possible to use the soluble recombinant selectin and the sugar chain as an active ingredient of the present invention.
   The agent of the present invention using the sugar chain as an active ingredient achieves a desired effect when administered to a living body in the form of an injectable solution or the like.
(f) Examples of the methods for inhibiting synthesis of sialyl Lewis x includes a method for inhibiting a function of a glycosyltransferase involved in biosynthesis of sialyl Lewis x or a method for preventing expression of the enzyme. Examples of the enzyme include an N-acetylgalactosaminyltransferase, a galactoslytransferase, an N-acetylglucosaminyltransferase, a fucosyltransferase, a sialyltransferase, and a sulfotransferase.
   Examples of the methods for inhibiting the function of the enzyme include competitive inhibition and rivalry inhibition by analogues (for example, benzyl-α-N-acetylgalactosamine, and the like) of bases of the enzymes (UDP-GalNAc (uridinediphosphate-N-acetylgalactosamine), UDP-Gal (uridinediphosphate-galactose), UDP-GlcNAc (uridinediphosphate-N-acetylglucosamine), UDP-Fuc (uridinediphosphate-fucose), UDP-SiaA (uridinediphosphate-sialic acid), and PAPS (active sulfate)).
   As the methods for preventing the expression of the enzyme include a method for introducing a nucleic acid having a nucleotide sequence encoding siRNA which causes RNA interference with a transcription product of a gene encoding the enzyme. The person skilled in the art can prepare the enzyme based on the nucleotide sequence of the nucleic acid encoding the enzyme for the purpose of the prevention of the expression and by appropriately selecting the nucleotide sequence of siRNA. It is possible to use the nucleic acid having the nucleotide sequence of siRNA as an active ingredient of the agent of the present invention.
   The agent of the present invention achieves an infiltration suppressing effect by introducing a nucleic acid prepared by an ordinary method into a virus vector employing a known method such as a lentivirus system (product of Invitrogen Corporation) and administering the virus vector to a living body to suppress the expression of sialyl Lewis x in subject cells.
(g) Examples of the induction of antibody dependent cell damaging activity include administration of an anti-CD43 antibody. Among anti-CD43 antibodies, an antibody humanized by recombining a modifiable region by a gene recombinant method (so-called humanized antibody) and a chimeric antibody are usable as an active ingredient for a therapeutic agent for leukemic cells. Since it has been revealed that the leukemic cells express CD43 according to the present invention, it is possible to cure the leukemia by administering the above-described antibody together with the recombinant leukemic cells (monocytes, macrophages, natural killer cells (NK cells), and the like) of which the expression of CD43 is suppressed by siRNA or the like to a living body. Namely, the antibody recognizes CD43 to bind to the leukemic cells and the recombinant leukemic cells which do not express CD43 by the recombination recognizes the antibody bound to the leukemic cells to exhibit cellular immunization effect on the antibody. By the cellular immunization effect, specifically killing of the leukemic cells is possible.
(h) A cell killing agent to which a cytotoxic agent is bound is expected to exhibit an efficient cell killing effect when administered followed by binding of such as an antimetabolite, an alkylation agent, anthracyclines, an antibiotic, an antimitotic agent, a chemotherapeutic agent, and a radioactive substance to a substance which has specific binding ability to CD43 glycoprotein such as anti-CD43 antibody. Further, specific examples of the cytotoxic agents include ricin, doxorubicin, daunorubicin, taxol, ethidium bromide, mitomycin, etoposide, tenoposide, vincristine, vinblastine, corticin, dihydroxyanthracindione, actinomycin D, 1-dehydrotestosterone, glucocorticoid, and the like. Also, it has been reported that CD43 is expressed in almost all cells other than mature B lymphocytes among the hematopoietic lineage cells. From these findings, a use of the cell killing agent for a bone marrow suppression before bone marrow transplant which is a great burden on patients due to irradiation and a megadose of an anticancer agent is also expected. That is, since administration of the killing agent enables specific elimination of hematopoietic lineage cells including hematopoietic stem cells, an application as a pretreatment agent for bone marrow transplant, which is reduced in burden on patients.

The method of suppressing the binding of selectin ligand sugar chain to selectin and the cell killing method targeting the selectin ligand sugar chain and the carrier protein retaining its sugar chain by using the agent of the present invention may preferably be performed by employing (a) methods for suppressing the expression of CD43 and selectin ligand sugar chain and (g) methods by induction of antibody dependent cell damaging activity by an anti-CD43 antibody and as the active ingredient of the agent of the present invention, the nucleic acid having the siRNA sequence which suppresses the expression of CD43 antigen and selectin ligand sugar chain, the anti-CD43 antibody, or the anti-selectin ligand sugar chain antibody, and, particularly, the nucleic acid having the siRNA sequence which suppresses the expression of CD43 antigen and selectin ligand sugar chain or the anti-CD43 antibody is particularly preferred.

It is possible to formulate the agent of the present invention in the form of a tablet, a capsule, a liquid, an injection, a granulation, a powder, an agent for lipo, an inhalant powder, and the like which depends on an administration route such as an oral administration and injection, an objective, a subject, and the like. A concentration of the active ingredient in the agent of the present invention is not particularly limited, and a preferred concentration is from 0.001% to 5%. For example, in the case where the agent of the present invention is used as a liquid for oral administration, the concentration may preferably be 0.01% (W/V) or more, most preferably 0.03% to 0.2% (W/V). In the case of an injectable solution for intramuscular or intravenous administration, the concentration may preferably be 0.01% (W/V) or more, most preferably 0.03% (W/V) or more. The agent of the present invention exhibits its pharmaceutical effect when administered directly to a vein. Therefore, the agent in the form that can be administered by injection, instillation, and the like is particularly preferable.

A dosage of the agent of the present invention is the matter to be decided independently in view of the administration route, the administration form, the intended purpose, a specific symptom, age, and weight of an animal of administration subject, and the like so that the effects of the present invention are most appropriately exhibited. Thus, the dosage is not particularly limited. For example, in the case of administration to human, the dosage is from about 1 to 1,000 mg per dose per adult, and the agent can be administered once to several times per day.

The agent of the present invention may contain an additive in addition to the active ingredient. Examples of the additive include salts, a preservative, an antibiotic, an anti-inflammatory agent, a mixture or combination of growth factors, and the like. Examples of the salts include sodium sulfate, sodium chloride, sodium hydrogenphosphate, and the like. Examples of the preservative include methyl paraoxybenzoate, propyl paraoxybenzoate, benzalkonium chloride, and the like. Examples of the antibiotic include oxytetracycline and the like. Examples of the anti-inflammatory agent include diclofenac and the like.

Also, a pharmaceutical aid which is used for ordinary medical compositions, such as filler and a stabilizing agent, may be used for the agent of the present invention without problem.

The agent of the present invention acts on suppression of infiltration of cells, and the "suppression" means "prevention" of preventing infiltration before occurrence of the infiltration, "prevention of aggravation" of preventing existing infiltration from further infiltration, and "improvement" of a state of infiltration. The agent of the present invention has a particularly excellent effect in prevention.

### 2. Method of the present invention

The method of the present invention is a cell infiltration suppression method which comprises suppressing the binding of selectin ligand sugar chain on cell surface to selectin and a cell killing method which targets at the carrier protein such as the CD43 protein retaining the selectin ligand sugar chain.

The suppression of binding of the selectin ligand on cell surface to selectin and the cell killing which targets at the selectin ligand sugar chain and CD43 retaining the selectin ligand sugar chain are achieved by methods that are described in the item of the agents of the present invention such as (a) methods for suppressing the expression of CD43; (b) methods for severing or inhibiting the binding between CD43 and sialyl Lewis x; (c) methods for binding to a complex of CD43 and sialyl Lewis x to cause a displacement of configuration; (d) methods for specifically decomposing CD43 or sialyl Lewis x, (e) methods for competitive inhibition or rivalry inhibition of the binding between sialyl Lewis x and selectin, (f) methods for inhibiting synthesis of sialyl Lewis x, (g) methods of induction of antibody dependent cell damaging activity by an antibody recognizing an extracellular domain of CD43, and (h) methods using a CD43 extracellular domain binding substance to which a cell damaging agent is bound.

Specific substances and methods of using the substances for realizing the above methods are as described in 1. The agent of the present invention.

### Examples

### (1) Confirmation of selectin ligand sugar chain expression in leukemic cells

Selectin ligand sugar chain expression was confirmed in pre-B lymphocytic leukemia cell strains (cell line established from pre-B lymphocytic leukemia patient-derived cells) KM3 cells (donated by Dr. Jun Minowada of Hayashibara Biochemical Laboratories, Inc.), NALL1 cells (donated by Prof. Isao Miyoshi of Hayashibara Biochemical Laboratories, Inc.), KOPN-K cells, LAZ-221 cells (donated by Dr. Shin Matsuo of Hayashibara Biochemical Laboratories, Inc.), and leukemic cells collected from acute pre-B lymphocytic leukemia patients (total number of specimens: 15), by using flow cytometer. As a negative control, cell strain Raji cells which are in the same B-cell line and proved to be free from the expression of selectin ligand sugar chain are used.

Fluorescence intensity and distribution of cell count of a sialyl Lewis x sugar chain antigen which is a major selectin ligand sugar chain were confirmed in flow cytometer by using a CSLEX antibody (antibody specifically recognizing the sialyl Lewis x structure; product of Becton, Dickinson and Company) to which a tandem dye APC-Cy7 was bound in accordance with a method described in J. Biol. Chem., 273, 41, 26779-26789 (1998). Rightward shifting in the histogram means an increase in the fluorescence intensity, i.e. an amount of expression of sialyl Lewis x.

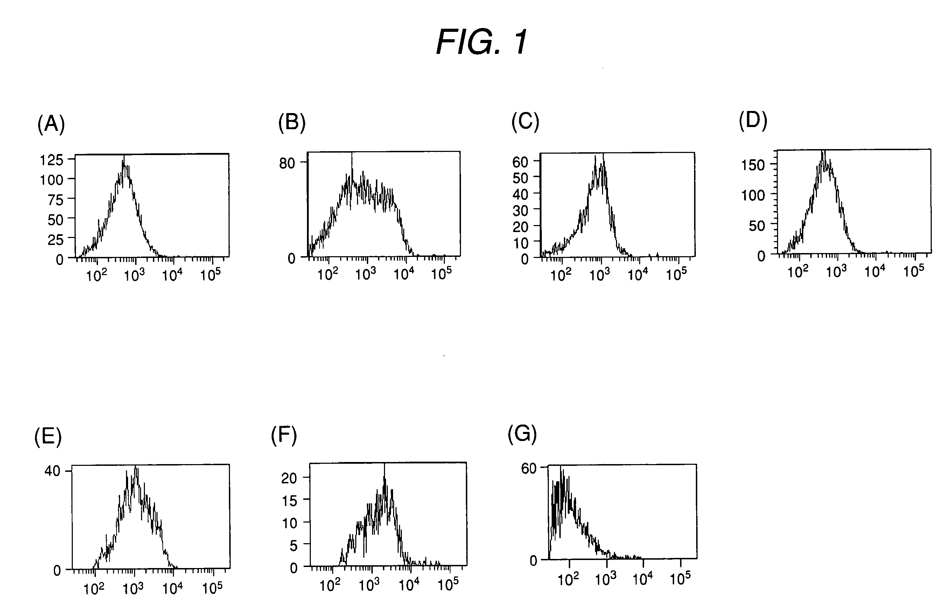

As a result, it was revealed that sialyl Lewis x is expressed at a high level in these cells (Fig. 1: (A) KM3 cell, (B) NALL1 cells, (C) KOPN-K cells, (D) LAZ-221 cells, (E) leukemia patient-derived leukemic cells #1, (F) leukemia patient-derived leukemic cells #2, (G) Raji cells).

Likewise, a binding ability to E-selectin and P-selectin was confirmed in flow cytometer through fluorescence intensity and distribution of cell count measured by using a recombinant protein (human chimera-Fc-Ig-E-selectin or human chimera-Fc-Ig-P-selectin; product of R & D Systems) in accordance with the method descried in Blood, 104, 3091-3096 (2004). Specifically, the recombinant E- and P- selectins which has a final concentration of 1 to 10 µg/mL were added to cells suspended to a phosphate buffered solution (PBS) to which 1 mmol/L of CaCl₂ and 1% serum had been added, and a reaction was conducted for 30 minutes on ice, followed by washing with PBS, thereby detecting a signal by a labeled antibody (product of Becton, Dickinson and Company) for human-Fc-Ig to which the tandem dye APC-Cy7 was bound. Rightward shifting in the histogram means an increase in the fluorescence intensity, i.e. binding ability to selectin.

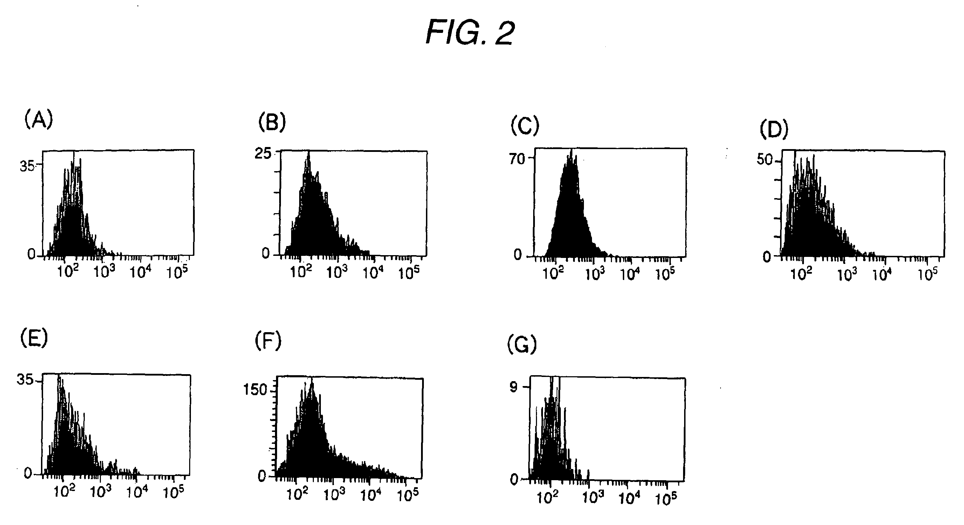

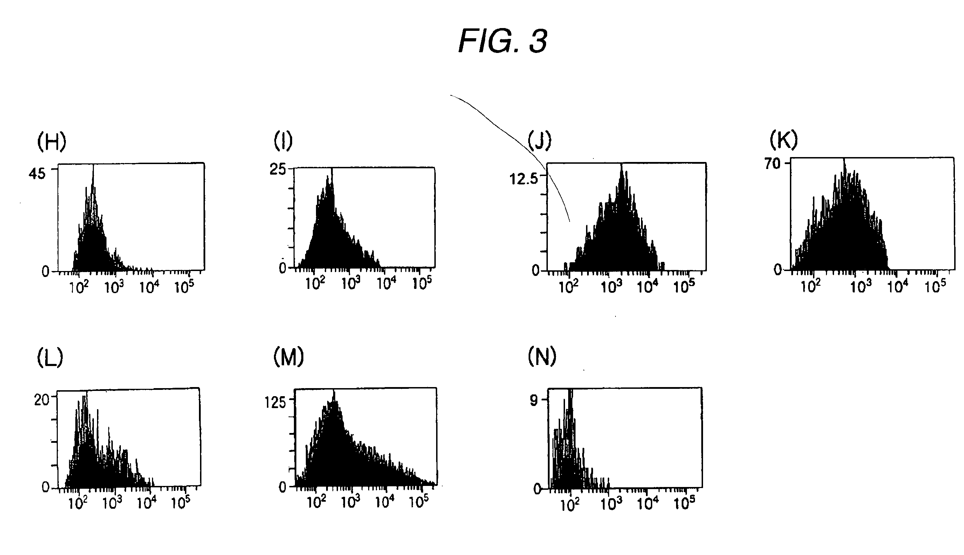

As a result, it was revealed that these cells have remarkable E-selectin and P-selectin binding abilities (Figs. 2 and 3). In Figs. 2 and 3, (A) and (H) are KM3 cell, (B) and (I) are NALL1 cells, (C) and (J) are KOPN-K cells, (D) and (K) are LAZ-221 cells, (E) and (L) are leukemia patient-derived leukemic cells #1, (F) and (M) are leukemia patient-derived leukemic cells #2, and (G) and (N) are Raji cells. (A) to (G) represent the E-selecting binding ability, and (H) to (N) represent P-selectin binding ability. Results of the 13 examples of leukemia patient-derived leukemic cells which are not described above were almost the same as the results of patient specimens shown in Figs. 1(E), 1(F), 2(E), 2(F), 3(L), and 3(M). The results show that the pre-B lymphocytic leukemia cells express the selectin ligand sugar chains on the cell surfaces.

### (2) Confirmation of selectin dependent cell adhesion ability in leukemic cell strain

In the course of adhesion of white blood cells flowing in a bloodstream to vascular endothelial cells, the binding mediated by the selectin ligand sugar chain expressed on a cell surface and selectin triggers the adhesion. A phenomenon of the white blood cells flowing as if rolling on the vascular wall with binding to selectin has been observed in the binding reaction, and such phenomenon is called white blood cell rolling. Since it is considered that the rolling occurs also in the case of the pre-B lymphocytic leukemia cells expressing the selectin ligand sugar chains in the initial step of the adhesion to vascular endothelial cells and tissue infiltration, it is possible to compare selectin-mediated cell adhesion abilities by flowing the pre-B lymphocytic leukemic cells on a cell strain expressing selectin and measuring cell rolling per unit time and unit area in vitro. The experiment was conducted in accordance with a method disclosed in J. Biol. Chem., 277, 3979-3984 (2002) by fixing of CHO cell strains (named CHO-E and CHO-P) expressing selectin on cell surfaces at a high density due to introduction of E- and P- selectin genes to a flow chamber, and then cells suspended in a 0.1% serum-added RPMI 1640 culture medium was introduced into the flow chamber at a concentration of 1 × 10⁶ cells/ml by using a syringe pump under a shearing stress of 1.0 dyne/cm². The cell rolling observed under a microscope was measured by using analysis software. The introduced cells were pre-B lymphocytic leukemic cell strain NALL1 cells, LAZ-221 cells, and Raji cells which were used as a negative control.

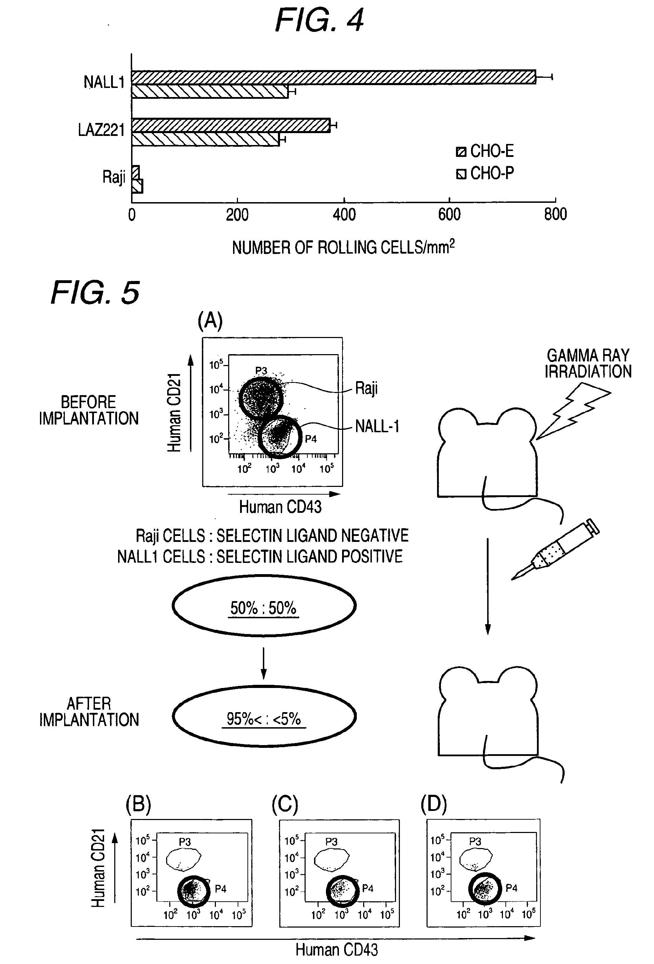

As a result, remarkable cell rolling and cell adhesion were observed in the pre-B lymphocytic leukemia cell strain expressing the selectin ligand and not in Raji cells (Fig. 4). From the result, it was suggested that the pre-B lymphocytic leukemia cells expressing selectin ligand has a selectin-dependent cell adhesion ability.

### (3) Confirmation of selectin dependent tissue migration of leukemic cells using immunodeficient mouse

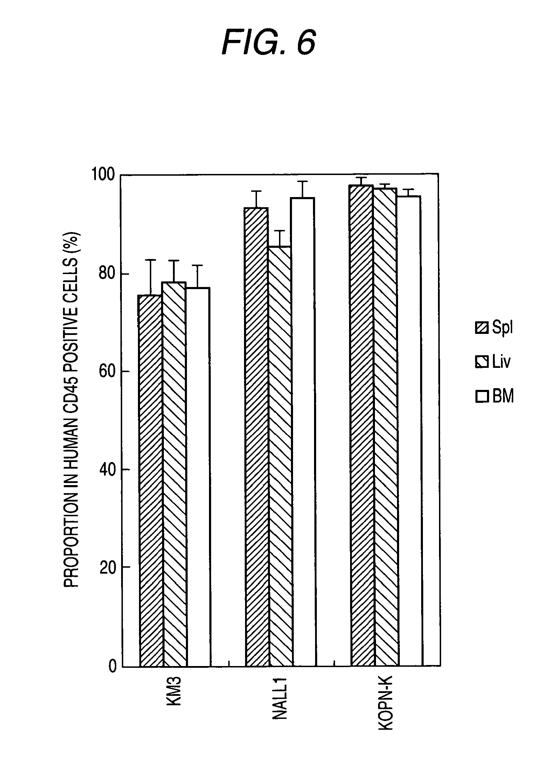

Involvement of cell adhesion ability via the selectin ligand and selectin in the tissue migration of leukemic cells was detected by using a mouse model. With reference to Blood, 103, 2900-2907 (2004), a selectin ligand positive pre-B leukemic cell strain (KM3, NALL1, or KOPN-K) was mixed with an equivalent amount of selectin ligand negative Raji cells to be introduced into a body of an irradiated immunodeficient mouse from the tail vein. Spleen cells, liver cells, and bone marrow cells were collected after 6 hours from the introduction, and introduced cells contained in the cells, i.e. the cells migrated to the tissue, were detected. The pre-B leukemic cells were surface antigen CD45 positive, CD43 positive, and CD21 negative, while Raji cells were CD45 positive, CD43 negative, and CD21 positive. Therefore, by using fluorescence labeled antibodies (APC-bound anti-human CD45 antibody, FITC-bound anti-human CD43 antibody, and PE-bound anti-human CD21 antibody; products of Becton, Dickenson and Company), the pre-B leukemic cells of CD45 positive, CD43 positive, and CD21 negative and the Raji cells of CD45 positive, CD43 negative, and CD21 positive were detected by using a flow cytometer. Experiment results of the cases of using the pre-B leukemic cells NALL1 cells and the Raji cells are shown in Fig. 5 as representative examples. Also, a summary of the experiment results of the 3 types of cells is shown in Fig. 6. Despite the cells were introduced in the equivalent amounts, an amount of the selectin ligand positive pre-B leukemic cells detected in each of the tissues was larger than that of the selectin ligand negative Raji cells. From the above findings, it was suggested that the migration of the leukemic cells to tissue is dependent on an expression level of the selectin ligand, and that the expression of selectin ligand sugar chain in the pre-B leukemic cells results in higher rates of the cell adhesion and the migration/infiltration to tissue.

### (4) Relationship between selectin ligand sugar chain and CD43 antigen

It was revealed that a cell surface antigen having the selectin ligand sugar chain which is the key for the tissue infiltration is only the CD43 antigen in pre-B lymphocytic leukemia cell strain NALL1 and the like.

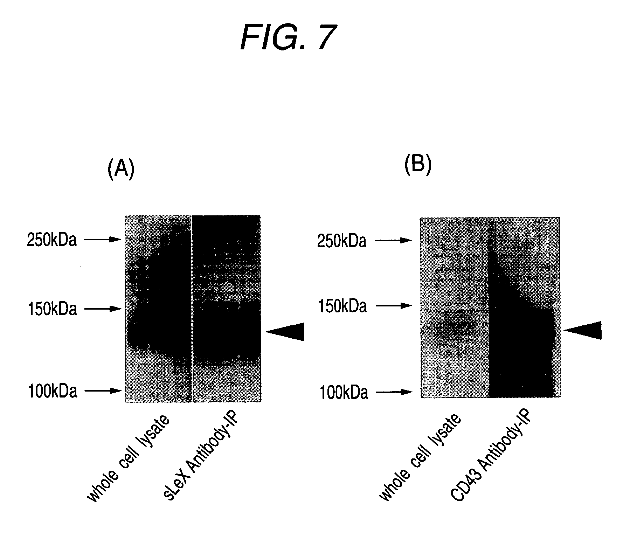

More specifically, by using an immunoprecipitation method, CSLEX1 which is an antibody for sialyl Lewis x, MEM59 which is an antibody for CD43 (product of Monosan), and protein G sepharose beads were added to a NALL1 cell lysate, followed by a reaction at 4°C overnight, and then the beads were collected by centrifugal separation to isolate proteins reacting to the each antibody. The proteins collected by the beads were dissolved into SDS-PAGE sample buffer, and the proteins each in a protein amount of 20 to 40 µg were subjected to electrophoresis in a 7.5% concentration polyacrylamide gel, followed by transfer to PVDF membrane. After that, Western blot analysis was conducted by MEM59 or CSLEX1. As a result, from the Western blotting according to MEM59 of the NALL1 cell lysate subjected to the immunoprecipitation by CSLEX1 (Fig. 7A), it was suggested that a protein of a molecular weight of a little less than 150 kDa was concentrated, and that the protein expressed by sialyl Lewis x was CD43. Likewise, from the Western blotting according to CSLEX1 of the NALL1 cell lysate subjected to the immunoprecipitation by MEM59 (Fig. 7B), it was suggested that a protein of a molecular weight of a little less than 150 kDa was concentrated, and sialyl Lewis x was expressed on the CD43 antigen. Since no signal was observed in regions other than the portion of the molecular weight of a little less than 150 kDa in the results of the Western blottings, it was suggested that almost all of proteins expressed by sialyl Lewis x were CD43.

### (5) Confirmation of suppression effects on selectin ligand sugar chain expression and selectin binding ability by inhibition of CD43 antigen expression

In pre-B lymphocytic leukemia cells, almost all of the selectin ligand sugar chains which are important for tissue infiltration exist on the CD43 antigen. Accordingly, study was conducted on a method for suppressing the tissue infiltration by reducing selectin-mediated cell adhesion ability through the suppression of CD43 antigen expression.

At first, siRNA for specifically suppressing the CD43 expression by RNA interference was designed. The sequence of the siRNA was designed by using siRNA sequence search software available on a homepage of Amibion Inc. (http://www.ambion.com/) and selecting several 19 to 21 bases at random from a CD43 gene sequence (J. Biol. Chem., 266, 13, 8483-8489 (1991)) (Table 1: a portion of the sequence indicated by thickened italic indicates the nucleotide sequence corresponding to the CD43 gene).

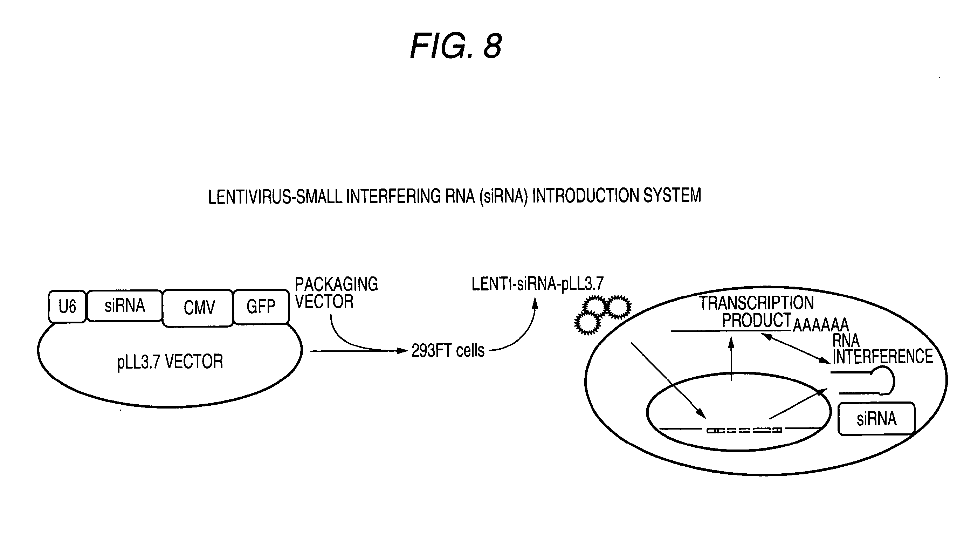

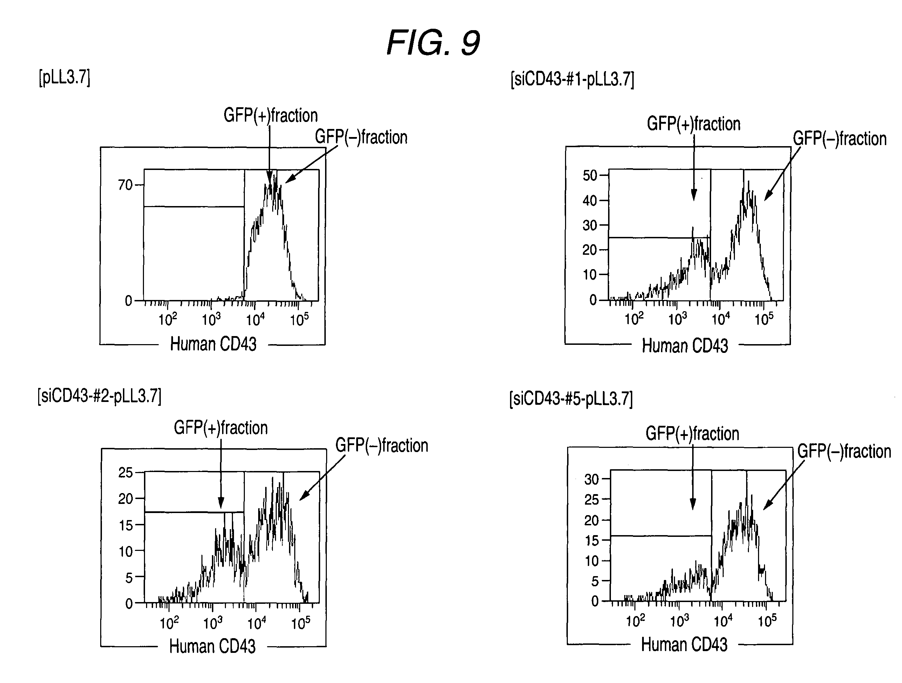

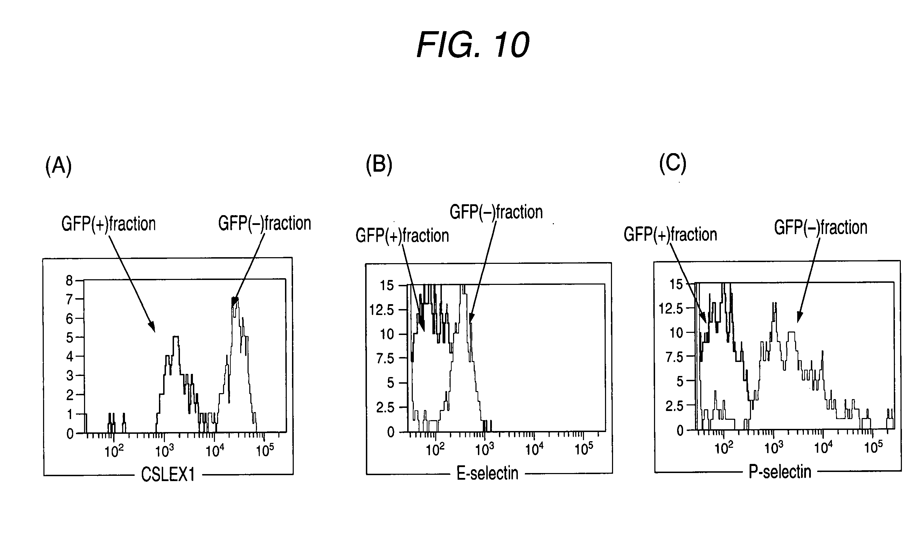

These siRNAs were expressed in cells to confirm the effects. For introduction of siRNA into cells, a lentivirus system (product of Invitrogen Corporation) which is able to gene introduction into blood cells at high efficiency and an expression vector for lentivirus pLL3.7 (Nat. Genet., 33, 401-406 (2003)) were used. In the pLL3.7 vector, it is possible to introduce a sense/antisense siRNA sequence including a hair-pin structure downstream of a U6 promoter. The pLL3.7 vector expresses Green Fluorescent Protein (GFP) by a CMV promoter at the same time. Accordingly, oligonucleotide including the sense chain, antisense chain, and hair-pin structure of the designed siRNA sequence (CD43-#1 to #5, CD43-$1 to $5; alphabet f at the end of the name indicates the sense chain, and r indicates the antisense chain) was chemically synthesized and then cloned to pLL3.7 vector, followed by introduction into 293 cells together with a vector for virus particle production. After that, siRNA expression lentivirus vector was collected from a supernatant of the culture medium (Fig. 8). Virus infection was caused by co-culture of the virus particles and NALL1 cells to introduce the siRNA into the cells and the siRNA is expressed. GFP was simultaneously expressed in the cells into which the siRNA was introduced by the pLL3.7 vector. Therefore, expression of the CD43 antigen and sialyl Lewis x and the selectin binding ability of the cells in which the GFP expression was observed, i.e. of the cells in which siRNA was expressed, were analyzed by using the antibody MEM59 (product of Monosan) for CD43, sialyl Lewis x antibody CSLEX1, and human chimera-Fc-Ig-E-or P-selectin with the use of a flow cytometer. As a result, in introduction of vacant vector (pLL3.7), a high rate of the CD43 expression was observed irrelevant from GFP positive or negative. On the other hand, in the cells into which the siRNA for CD43 was introduced (siCD43, #1, #2, #5-pLL3.7), significant reduction of CD43 expression was observed in the GFP positive fraction, i.e. in the cells into which the siRNA was introduced (Fig. 9). Likewise, remarkable reductions in sialyl Lewis x expression and selectin binding ability were observed (Fig. 10).
[Table 1]
Table 1: siRNA sequence for CD43 antigen (6) Confirmation of suppression effect on the binding between leukemic cells and selectin by selectin ligand sugar chain

### synthesis inhibition

Benzyl-α-N-acetylgalactosamine (Bz-α-GalNAc; product of Sigma-Aldrich Japan, K.K.) which is one of sugar chain synthesis inhibiting agents was added to a culture supernatant of NALL1 cells to detect its binding ability to selectin. Benzyl-α-N-acetylgalactosamine is subject to sugar chain modification like N-acetylgalactosamine added to serine and threonine of a protein when taken into a cell. Therefore, since the original sugar chain modification to a glycoprotein is suppressed by an addition of an excessive amount of benzyl-α-N-acetylgalactosamine, benzyl-α-N-acetylgalactosamine is used as an O-linked sugar chain synthesis inhibiting agent. After adding 4 mM of benzyl-α-N-acetylgalactosamine to the culture supernatant of NALL1 cells, expressions of the CD43 antigen and sialyl Lewis x and binding ability to E-and P-selectin were detected by a flow cytometer after 3 day-culture.

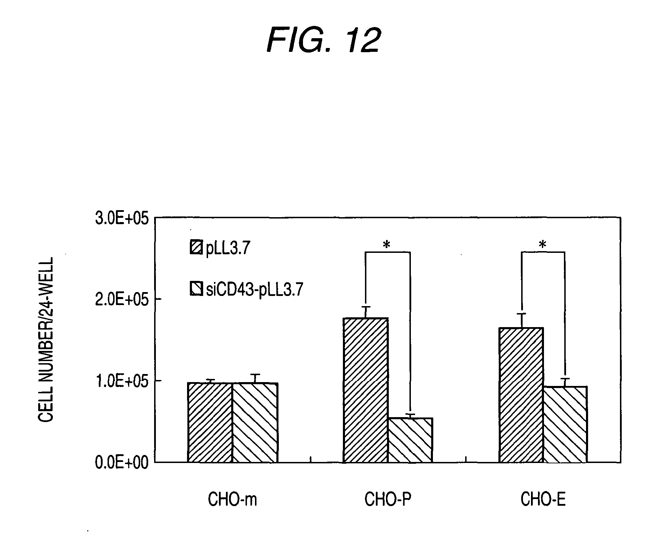

As a result, though no change was observed in the expression of CD43 antigen, the sialyl Lewis x expression was remarkably reduced and the selectin binding ability was almost lost (Fig. 12). Therefore, it was suggested that the addition of benzyl-α-N-acetylgalactosamine to NALL1 cells suppresses the expression of sialyl Lewis x which is synthesized on the O-linked sugar chain on the CD43 antigen, reduces the binding abilities to E- and P-selectins, and is effective for suppression of infiltration of leukemic cells via the adhesion to vascular endothelial cells. Likewise, it is expected that similar effects are exhibited by an activity inhibiting agent for glycosyltransferase which synthesizes the selectin ligand sugar chain on the O-bound sugar chain of the CD43 antigen and sugar chain synthesis inhibition by analogues obtainable by various modifications on a sugar chain or a sugar nucleotide that are ingredients of the activity inhibiting agent.

### (7) Confirmation of disappearance of binding ability of leukemic cells to selectin by decomposition of CD43 antigen

Expressions of CD43 antigen and selectin ligand sugar chain and E- and P-selectin binding abilities by O-sialoglycoprotein endopeptidase (OSGPEP; product of Cedarlane Laboratories Ltd.) which selectively decomposes a mucin protein which is rich in sialic acid were studied. To 500 µL of 10% serum-added RPMI 1640 culture liquid in which 2.5×10⁵ NALL1 cells were suspended, 50 µL of 0.2 mg/mL of OSGPEP was added every 24 hours for 3 consecutive days, followed by detection of the expressions of CD43 antigen and sialyl Lewis x and the binding abilities to selectins with the use of a flow cytometer.

As a result, disappearance of the expression of CD43 antigen and the sialyl Lewis x by the OSGPEP treatment were observed, and it was revealed that the binding abilities to E- and P-selectins were almost disappeared (Fig. 12). The CD43 antigen protein is rich in serine and threonine to which the O-linked sugar chain is added. In the pre-B lymphocytic leukemia cells, it is considered that a decomposition reaction was proceeded by OSGPEP since sialyl Lewis x containing sialic acid exists at a terminus of the O-linked sugar chain which is added to serine and threonine. Therefore, it was suggested that decomposition and digestive treatment of the CD43 antigen containing the selectin ligand sugar chain which is expressed in the pre-B lymphocytic leukemia cells provide suppression of the selectin-mediated adhesion to vascular endothelial cells and are effective for the suppression of infiltration of leukemic cells.

### (8) Effects of suppression on rolling and cell adhesion of leukemic cells by the agent of the present invention

Rolling and cell adhesion ability of KM3 cells into which a vector (siCD43-pLL3.7) in which the siRNA for CD43 was introduced were detected. As a result, as shown in Fig. 12, in the cells into which the siRNA for CD43 was introduced, rolling and cell adhesion abilities to CHO-E and CHO-P cells were remarkably reduced as compared to vacant vector-introduced cells (pLL3.7). Since the effect of the reduction is correlated to the cell surface selectin ligand expression level shown in Fig. 10, it was suggested that the leukemic cells treated by the methods described in (5) to (7) reduce the selectin dependent cell adhesion ability.

### (9) Effect of suppression of leukemic cell migration to organs by the agent of the present invention

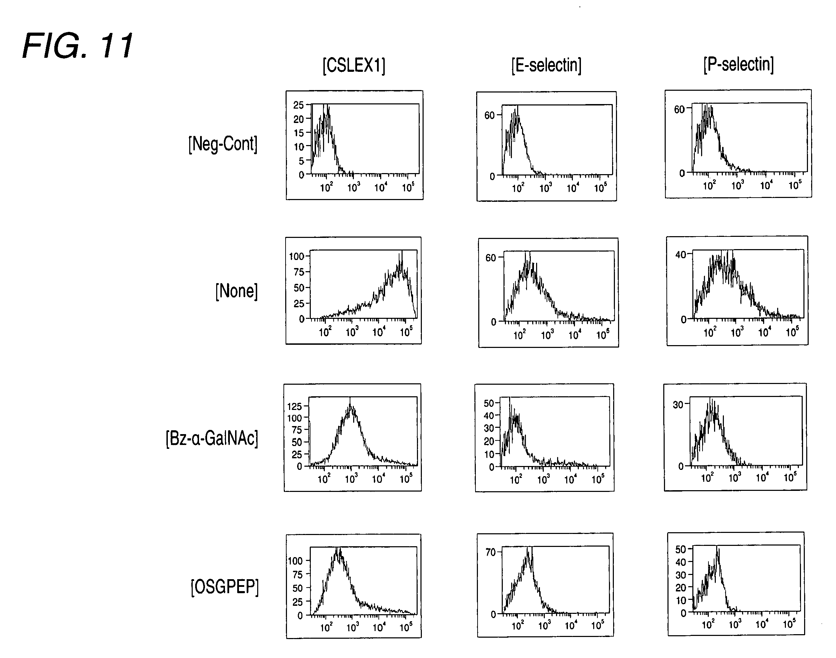

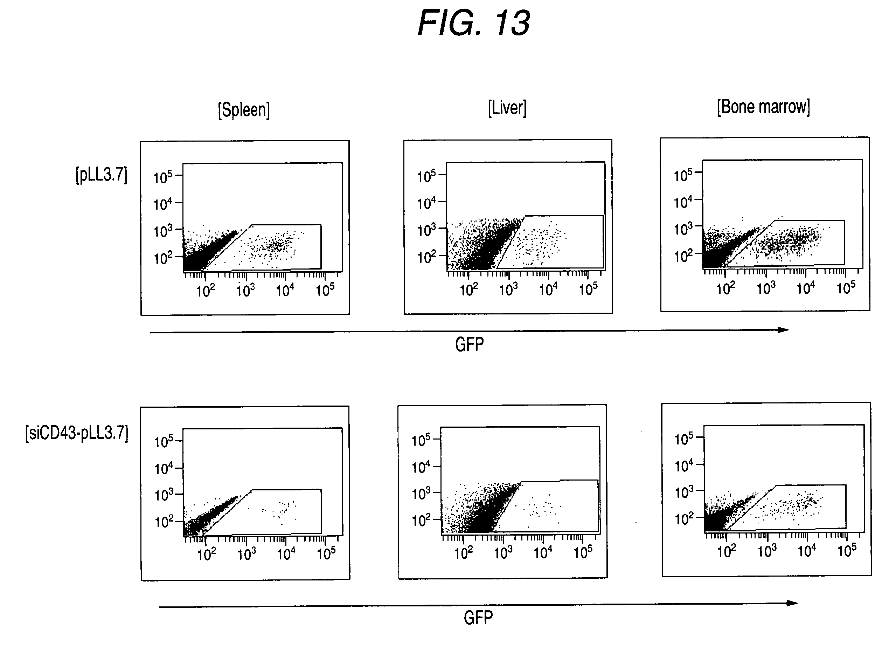

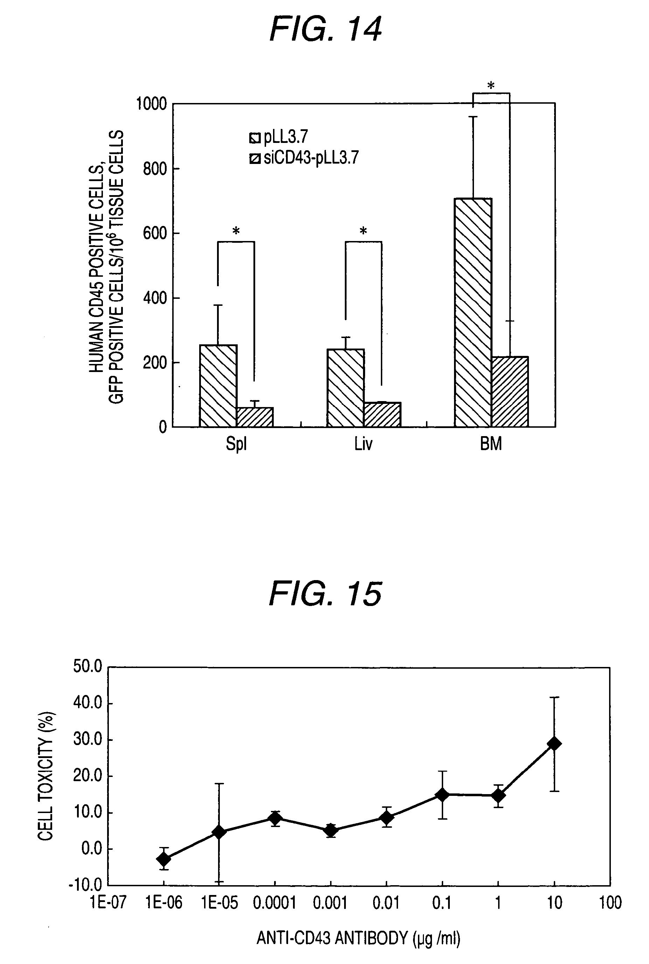

Organ migration ability of cells treated with the agent of the present invention was detected. Each of a vacant vector (pLL3.7) free from the siRNA expression and a vector (siCD43-pLL3.7) in which the siRNA for CD43 was introduced into NALL1 cells to be administered to an irradiated immunodeficient mouse from the tail vein. Spleen cells, liver cells, and bone marrow cells were collected from each of the mice after 6 hours from the administration, and introduced cells which had transferred to the tissue were detected among the cells. Since GFP was introduced in both of the vacant vector and the vector in which siRNA was introduced, the numbers of human CD45 positive cells and GFP positive cells in the collected cells were compared. In Fig. 14, one example of a flow cytometric analysis result of the tissue after the cell introduction is shown. Also, in Fig. 15, a summary of the experiment results is shown. As a result, a remarkable reduction in the number of cells transferred to the tissue was observed in the cells into which the siRNA for CD43 was introduced as compared to the vacant vector introduced-cells. Since the result is correlated to the cell surface selectin ligand expression level shown in Fig. 11 and the selectin dependent cell adhesion ability shown in Fig. 13, it was suggested that the leukemic cells treated by the methods described in (5) to (7) decrease the migration to tissue, i.e. the tissue infiltration, in accordance with the selectin ligand expression level.

### Confirmation of life prolongation effect on mouse

It is possible to confirm a mouse life prolongation effect by the agent of the present invention.

More specifically, about 5×10⁵ untreated NALL1 cells or NALL1 cells treated by the methods described in (5) to (7) were administered to immunodeficient mice irradiated with radioactive rays from 350 to 400 cGy from the tail vain, and the mice were reared in an SPF room by feeding sterilized water and sterilized diet. After about 2 months of the rearing, survival rates of the mice was measured. Liver, spleen, kidney, and the like of each of the mice were removed when the mice died or euthanized after the 2 month-rearing to create frozen sections, and then blood cells infiltrated the tissues were observed by a hematoxylin-eosin staining method.

Also, peripheral blood and bone marrow aspirate were collected from each of the mice to detect a proportion of the leukemic cells in a white blood cell fraction by using the human CD45 antibody in accordance with an ordinary method with the use of a flow cytometer. It is considered that the implanted leukemic cells were hardly observed in the each organ, peripheral blood, and bone marrow aspirate of the mice in which the NALL1 cells treated by the method described in (5) to (7) were implanted. Further, a high survival rate was observed as the effect of tissue infiltration suppression.

### (11) Measurement of antibody dependent cytotoxic activity

An antibody dependent cytotoxic activity with the use of the anti-CD43 antibody was measured. Study was conducted by using NALL1 cells as target cells. The cells were adjusted to 2×10⁵ cells/ml in 5% fetal calf serum (product of Gibco BRL) containing RPMI 1640 culture medium (product of Gibco BRL). 50 µl of the cells were added to 96-well U-bottom plate (product of Falcon), followed by addition of an antibody for measuring the antibody dependent cytotoxic activity (mouse anti-human CD43 antibody, MEM59, DFT-1) and 50 µl of effecter cells (mouse or human mononuclear cells). After incubation in 37°C CO₂ incubator for 4 hours, 50 µl of a culture supernatant was transferred to a 96-well flat bottom plate (product of Falcon), and 50 µl of Substrate Mix (product of Promega Corporation) was added to each of the wells, followed by incubation at a room temperature under shading for 30 minutes. After that, 50 µl of Stop Solution (product of Promega Corporation) was added, and then 490 nm absorbance of each of the wells was measured by a measurement system VERSA max (product of Molecular Devices Corporation). The cell damaging activity (%) was calculated by (A-B-C)/(D-C) × 100. In the calculation formula, A means absorbance when the target cells were incubated under the presence of antibody and effecter cells; B means absorbance when only the effecter cells were incubated; C means absorbance when only the target cells were incubated, and D means absorbance in the case where the target cells were subjected to maximum toxicity. As a result, an antibody concentration dependent cytotoxic activity was detected, and the maximum value thereof was about 30%. From the above findings, it was suggested that the anti-CD43 antibody is effective also as a cell killing agent (Fig. 16).

Although the present invention has been described in detail with reference to specific examples in the foregoing, it is apparent to person skilled in the art that it is possible to add various alterations and modifications insofar as the alterations and the modifications do not deviate from the sprit and scope of the present invention.

This patent application is based on Japanese Patent Application No. 2004-184583 filed on June 23, 2004, and the contents thereof are incorporated herein by reference.

### Industrial Applicability

The present invention provides an agent for effectively suppressing intercell adhesion and tissue infiltration/metastasis, a method for suppressing tissue infiltration/metastasis, an agent for killing hematopoietic lineage cells including hematopoietic malignant tumor cells, and a cell killing method.

### Brief Description of the Drawings

[Fig. 1] Diagrams which show results of analyses of sialyl Lewis x expression status using a flow cytometer, wherein the results are A: KM3, B: NALL1, C: KOPN-K, D: LAZ221, E: pre-B leukemia patient (#1), F: pre-B leukemia patient (#2), and G: reaction between Raji cells and CSLEX1 (sialyl Lewis x antibody labeled with APC-Cy7). As used herein, Raji cells is B cell line cell strain free from selectin ligand expression and used as a negative control of this experiment. The horizontal axis represents fluorescence intensity of the APC-Cy7-labeled CSLEX1, and the vertical axis represents cell count.
[Fig. 2] Diagrams which show results of analyses of binding abilities to human chimera-Fc-Ig-E-selectins using a flow cytometer. Each (A) to (G) shows a reaction between human chimera-Fc-Ig-E-selectins (A to G) labeled with APC-Cy7 and A: KM3, B: NALL1, C: KOPN-K, D: LAZ221, E: pre-B leukemia patient (#1), F: pre-B leukemia patient (#2), and G: Raji cells. Raji cells were used as a negative control. The horizontal axis represents fluorescence intensity of the APC-Cy7-labeled E-selectin, and the vertical axis represents cell count.
[Fig. 3] Diagrams which show results of analyses of binding abilities to human chimera-Fc-Ig-P-selectins using a flow cytometer. Each (H) to (N) shows reaction between human chimera-Fc-Ig-P-selectins (H to N) labeled with APC-Cy7 and H: KM3, I: NALL1, J: KOPN-K, K: LAZ221, L: pre-B leukemia patient (#1), M: pre-B leukemia patient (#2), and N: Raji cells. Raji cells were used as a negative control. The horizontal axis represents fluorescence intensity of the APC-Cy7-labeled P-selectin, and the vertical axis represents cell count.
[Fig. 4] A diagram which shows a result of analysis of the number of cells rolling on CHO cells (named CHO-E and CHO-P) in which E-selectin and P-selectin of pre-B leukemic cells were excessively expressed. Raji cells were used as a negative control. The horizontal axis represents the number of rolling cells per unit (mm²).
[Fig. 5] Figures which show an experiment for detecting selectin dependent tissue migration by introducing a cell suspension containing equal amounts of NALL1 cells and Raji cells into an immunodeficient mouse and a diagram which shows results of analyses by a flow cytometer before and after the cell introduction. Each A to D is A: NALL1/Raji cell mixture before introduction; B: spleen of cell-introduced mouse; C: liver of cell-introduced mouse; and D: bone marrow cells of cell-introduced mouse. The horizontal axis and the vertical axis represent fluorescent intensity of fluorescence-labeled CD43 antibody and fluorescence-labeled CD21 antibody.
[Fig. 6] A diagram which shows a summary of the results of experiments for detecting selectin dependent tissue migration using immunodeficient mouse. Proportions of KM3, NALL1, and KOPN-K cells in human CD45 positive cells in the tissues are shown. Each Spl, Liv, and BM shows a result in spleen, liver and bone marrow cells.
[Fig. 7] Diagrams (pictures) which show results of immunoprecipitation-Western blotting analyses using a NALL1 cell lysate. (A) is the diagram of analysis of the western blotting with CD43 antibody MEM59 of a cell lysate immunoprecipitated by CSLEX1, and (B) is the diagram of analysis of the western blotting with CSLEX1 of an immunoprecipitated cell lysate by CD43 antibody MEM59. "Whole cell lysate" indicates a whole cell lysate before the immunoprecipitation, and "sLeX antibody-IP" and "CD43 antibody-IP" indicate results of samples which is immunoprecipitated by sLeX antibody CSLEX1 and CD43 antibody MEM59.
[Fig. 8] A schematic diagram which shows an siRNA introduction system by lentivirus. With a packaging vector, siRNA introduced in pLL3.7 vector was introduced into 293FT cells. The cells were transmitted with the thus-produced virus vector to introduce and express siRNA.
[Fig. 9] Diagrams which show flow cytometry analysis results of CD43 expression in siRNA introduced-NALL1 cells. The horizontal axis represents fluorescence intensity of the fluorescence labeled CD43 antigen, and the vertical axis represents the cell count. Also, the GFP(+) fraction and the GFP(-) fraction show a fraction of GFP expression positive cells, i.e. cells into which the virus vector was introduced and a fraction of GFP expression negative cells, i.e. cells into which the virus vector was not introduced. Further, [pLL3.7] indicates results obtained by using a vacant vector free from siRNA expression, [siCD43-#1-pLL3.7], [siCD43-#2-pLL3.7], and [siCD43-#5-pLL3.7] indicate results obtained by using vectors in which siRNA for CD43 was introduced.
[Fig. 10] Diagrams which show flow cytometer analysis results of sialyl Lewis x expression and E- and P-selectin binding abilities of the siRNA-introduced NALL1 cells. The analysis result of sialyl Lewis x expression of NALL1 cells into which siRNA#2 for CD43 was introduced is shown in (A); the analysis result of binding ability of E-selectin is shown in (B); and the analysis results of the binding ability to P-selecting is shown in (C). The horizontal axes represent the APC-Cy7-labeled CSLEX1 antibody, E-selectin fluorescent intensity, and P-selectin fluorescent intensity, while the vertical axis represents the cell counts.
[Fig. 11] Diagrams which show analysis results of sialyl Lewis x expression and selectin binding abilities of NALL1 cells treated with benzyl-α-N-acetylgalactosamine (Bz-α-GalNAc) and OSGPEP obtained by using a flow cytometer. The horizontal axes represent the APC-Cy7-labeled CSLEX1 antibody, E-selectin fluorescent intensity, and P-selectin fluorescent intensity, while the vertical axis represents the cell counts. [CSLEX1] and [E-selectin]/[P-selectin] indicate the sialyl Lewis x expression and E-selectin/P-selectin binding ability. [Neg-Cont] indicates the analysis result when a primary antibody of non-treated cells was absent; [None] indicates the analysis result of the non-treated cells; [Bz-α-GalNAc] indicates the analysis result of the cells treated with benzyl-α-N-acetylgalactosamine; and [OSGPEP] indicates the analysis result of the cells treated with OSGPEP.
[Fig. 12] Results of analyses of the numbers of cells rolling on CHO cells in which E-selectin and P-selectin of KM3 cells into which siRNA for CD43 was introduced were excessively expressed. Each of the results is an average value of 3 experiments. Represented by * is the case wherein the P value was 0.05 or less which has a significant statistical difference. CHO-m indicates the result of CHO cells into which a vacant vector was introduced.
[Fig. 13] Diagrams which shows results of analyses by a flow cytometer of cells of tissues of an immunodeficient mouse in which siRNA-introduced NALL1 cells for CD43 was implanted. The horizontal axis represents fluorescent intensity of GFP, and the vertical axis represents florescence intensity by a fluorescence dye PE. By using the NALL1 cells into which a vacant vector free from the siRNA expression was introduced and a vector in which siRNA for CD43 was introduced, [pLL3.7] and [siCD43-pLL3.7] are obtained. Each of [Spleen], [Liver], and [Bone marrow] indicates the result in spleen cells, the result in liver cells, and the result in bone marrow cells.
[Fig. 14] A diagram which shows a summary of the results of analyses by a flow cytometer of cells of tissues of an immunodeficient mouse in which siRNA-introduced NALL1 cells for CD43 were implanted. Average values obtained by conducting 3 experiments on each of the numbers of human CD45 cells and GFP positive cells per 10⁶ cells of each of the tissues are shown. Represented by * is the case wherein the P value was 0.05 or less which represents a significant statistical difference. In the horizontal axis, Spl, Liv, and BM indicate results in spleen cells, liver cells, and bone marrow cells. The vertical axis indicates the number of human CD45 positive cells and GFP positive cells per 10⁶ cells of each of the tissues.
[Fig. 15] A result of measurement of an antibody dependent cytotoxic activity obtained when the anti-CD43 antibody and the effecter cells were added to NALL1 cells. The horizontal axis represents an anti-CD43 antibody concentration, and the vertical axis represents cell toxicity (%).

## Claims

1. An infiltration suppressor which suppresses binding between a selectin ligand sugar chain on cell surface and selectin.

2. The infiltration suppressor according to claim 1, which comprises a nucleic acid comprising a small-interfering RNA (siRNA) sequence as an active ingredient.

3. The infiltration suppressor according to claim 2, wherein the small-interfering RNA (siRNA) sequence comprises any one of the nucleotide sequences of SEQ ID NOs:1 to 20.

4. The infiltration suppressor according to claim 1, which comprises endo-O-glycosidase as an active ingredient.

5. The infiltration suppressor according to claim 1, which comprises an anti-CD43 antibody as an active ingredient.

6. The infiltration suppressor according to claim 1, which comprises at least one sugar chain selected from the group consisting of sugar chains having human-Fc-Ig-chimera E-selectin, human-Fc-Ig-chimera P-selectin, and a sialyl Lewis x structure as an active ingredient.

7. Use of the infiltration suppressor according to any one of claims 1 to 6 for preventing tissue infiltration of cells.

8. An infiltration suppressing method, which comprises suppressing binding between a selectin ligand sugar chain on cell surface and selectin.

9. The method according to claim 8, which comprises suppressing addition of the selectin ligand sugar chain to cell surface antigen CD43.

10. The method according to claim 8, which comprises suppressing expression of the cell surface antigen CD43.

11. The method according to claim 8, which comprises suppressing expression of a glycosyltransferase gene adding the selectin ligand sugar chain to the cell surface antigen CD43.

12. The method according to claim 11, wherein the glycosyltransferase gene is a gene encoding an N-acetylgalactosaminyltransferase, a gene encoding a galactosyltransferase, a gene encoding an N-acetylglucosaminyltransferase, a gene encoding a fucosyltransferase, a gene encoding a sialyltransferase, or a gene encoding a sulfotransferase.

13. The method according to any one of claims 8 to 12, which comprises using a nucleic acid comprising a small-interfering RNA (siRNA) sequence.

14. The method according to claim 13, wherein the small-interfering RNA (siRNA) sequence comprises any one of the nucleotide sequences of SEQ ID NOs:1 to 20.

15. The method according to claim 8, which comprises severing the binding between the cell surface antigen CD43 and the selectin ligand sugar chain.

16. The method according to claim 8, which comprises decomposing the cell surface antigen CD43.

17. The method according to claim 8, which comprises decomposing the selectin ligand sugar chain.

18. Use of a nucleic acid comprising a small-interfering RNA (siRNA) sequence for suppressing of binding between a selectin ligand sugar chain and selectin.

19. A cell killing agent which binds to a selectin ligand sugar chain on a cell surface and a carrier protein retaining the sugar chain to induce cell death specifically on cells expressing a glycoprotein.

20. The cell killing agent according to claim 19, wherein the carrier protein retaining the selectin ligand sugar chain is a CD43 protein.

21. The cell killing agent according to claim 19, wherein a substance binding to the carrier protein is an antibody or a peptide specifically binding to an epitope in an extracellular region of the CD43 protein.

22. The cell killing agent according to claim 21, to which a cytotoxic agent is bound.

23. The cell killing agent according to claim 22, wherein the cytotoxic agent is selected from the group consisting of antimetabolites, alkylation agents, anthracyclines, antibiotics, antimitotic agents, chemotherapeutic agents, and radioactive substances.

24. The cell killing agent according to claim 22, wherein the cytotoxic agent is selected from the group consisting of ricin, doxorubicin, daunorubicin, taxol, ethidium bromide, mitomycin, etoposide, tenoposide, vincristine, vinblastine, corticin, dihydroxyanthracindione, actinomycin D, 1-dehydrotestosterone, and glucocorticoid.

25. The cell killing agent according to claim 21, wherein the antibody specifically binding to the epitope in the extracellular region of the CD43 protein is a human monoclonal antibody, a synthetic antibody, a polyclonal antibody, or a chimeric antibody.

26. A cell killing method, comprises binding the cell killing agent according to claims 19 to 25 to a selectin ligand sugar chain on a cell surface and a carrier protein retaining the sugar chain.

27. The method according to claim 26, wherein the cell is hematopoietic lineage cell including a hematopoietic malignant tumor cell and a hematopoietic stem cell expressing CD43.
